(19) Europäisches Patentamt
European Patent Office
Office européen des brevets

(11) **EP 1 671 614 B1**

(12) **FASCICULE DE BREVET EUROPEEN**

(45) Date de publication et mention
de la délivrance du brevet:
**02.06.2010 Bulletin 2010/22**

(51) Int Cl.:
*A61K 8/04* *(2006.01)*        *A61K 8/25* *(2006.01)*
*A61K 8/92* *(2006.01)*        *A61Q 1/00* *(2006.01)*
*A61Q 19/00* *(2006.01)*       *A61Q 19/08* *(2006.01)*

(21) Numéro de dépôt: **05292536.9**

(22) Date de dépôt: **30.11.2005**

(54) **Emulsion cosmétique comprenant des particules solides**

Kosmetische Emulsion enthaltend feste Partikeln

Cosmetic emulsion comprising solid particles

(84) Etats contractants désignés:
**AT BE BG CH CY CZ DE DK EE ES FI FR GB GR
HU IE IS IT LI LT LU LV MC NL PL PT RO SE SI
SK TR**

(30) Priorité: **17.12.2004 FR 0453045**

(43) Date de publication de la demande:
**21.06.2006 Bulletin 2006/25**

(73) Titulaire: **L'Oréal
75008 Paris (FR)**

(72) Inventeurs:
• **Themens, Agnès
92340 Bourg la Reine (FR)**

• **Arnaud, Pascal
94240 L'Hay les Roses (FR)**

(74) Mandataire: **Boulard, Denis
L'Oréal
D.I.P.I.
25-29 Quai Aulagnier
92600 Asnières (FR)**

(56) Documents cités:
**EP-A- 1 325 729        EP-A- 1 352 626
FR-A- 2 774 587        FR-A- 2 843 024
FR-A- 2 860 156**

**Description**

La présente invention a pour objet une composition cosmétique de maquillage ou de soin pour la peau y compris les lèvres sous forme d'émulsion.

La composition de maquillage selon l'invention peut être un fond de teint, notamment à appliquer sur le visage ou le cou, un produit anti-cernes, une crème teintée, une composition de maquillage du corps, un fard à paupières, un fard à joues, ou un rouge à lèvres.

La composition de soin peut être un produit de soin de la peau tels qu'une base de soin pour la peau, une crème de soin (crème de jour, de nuit, anti-rides), une base de maquillage, une composition de soin pour les lèvres (baume à lèvres).

Au cours du processus de vieillissement, il apparaît différents signes sur la peau, se traduisant notamment par une modification de la structure et des fonctions cutanées. Les principaux signes cliniques du vieillissement cutané sont notamment l'apparition de ridules et de rides profondes, en augmentation avec l'âge. On constate en particulier une désorganisation du "grain" de la peau, c'est-à-dire que le micro-relief est moins régulier et présente un caractère anisotrope.

Il est connu de traiter ces signes du vieillissement en utilisant des compositions cosmétiques ou dermatologiques contenant des actifs capables de lutter contre le vieillissement. Ces actifs agissent sur les rides en éliminant les cellules mortes de la peau et en accélérant le processus de renouvellement cellulaire. Toutefois, ces actifs présentent l'inconvénient de n'être efficaces pour le traitement des rides qu'après un certain temps d'application. Or, on cherche de plus en plus à obtenir un effet immédiat des actifs utilisés.

D'autres méthodes connues pour camoufler les défauts de la peau utilisent des compositions contenant des charges dites à effet de flou ou « soft-focus », comme il est décrit dans la demande de brevet EP-A-1099437. Ces compositions atténuent par effet optique les défauts cutanés tels que les taches, les rides, les ridules.

Toutefois, les résultats obtenus avec ces compositions sont souvent insuffisants pour atténuer voire masquer efficacement les rides les plus prononcées.

En outre, les particules solides comme les charges, les matières colorantes pulvérulentes présentes dans les compositions de maquillage de la peau présentent l'inconvénient de se concentrer dans les rides, en particulier les rides profondes, accentuant ainsi les irrégularités de la peau. L'application de ces compositions sur des peaux particulièrement ridées (notamment les peaux matures) conduit à un maquillage marquant ou révélant les rides.

Il existe donc un besoin pour une composition de maquillage ou de soin de la peau permettant d'obtenir un camouflage satisfaisant des défauts de la peau, notamment les rides, les ridules, les taches et plus particulièrement les rides.

Les inventeurs ont mis en évidence qu'il était possible d'obtenir une telle composition par l'introduction de particules choisies, notamment selon leur taille et/ou leur nature chimique, dans la phase huileuse et dans la phase aqueuse d'une émulsion.

L'invention a pour objet, selon un premier aspect, une composition cosmétique de maquillage ou de soin de la peau sous forme d'émulsion comprenant une phase aqueuse et une phase huileuse, caractérisée par le fait qu'elle comprend une dispersion de particules de cire de taille moyenne en volume inférieure ou égale à 1 $\mu$m dans la phase aqueuse et des particules de taille moyenne en volume inférieure ou égale à 15 $\mu$m dispersée dans la phase huileuse.

L'invention a encore pour objet une composition cosmétique de maquillage ou de soin de la peau sous forme d'émulsion comprenant une phase aqueuse et une phase huileuse, caractérisée par le fait qu'elle comprend une dispersion d'un composé sous forme de particules de taille moyenne en volume inférieure ou égale à 1$\mu$m dans la phase aqueuse et une dispersion de particules du même composé de taille moyenne en volume inférieure ou égale à 50 $\mu$m dans la phase huileuse.

Selon un mode de réalisation préféré de l'invention, les particules de taille moyenne en volume inférieure ou égale à 1$\mu$m dispersées dans la phase aqueuse sont choisies parmi les cires, les composés minéraux et leurs mélanges.

L'invention a également pour objet une composition cosmétique de maquillage ou de soin de la peau sous forme d'émulsion comprenant une phase aqueuse et une phase huileuse, caractérisée par le fait qu'elle comprend des particules de taille moyenne en volume inférieure ou égale à 1$\mu$m choisies parmi des cires, des particules de silice, et leurs mélanges, dispersées dans la phase aqueuse et des particules choisies parmi les poudres de cire, les poudres de silice, et leurs mélanges dispersées dans la phase huileuse.

Selon un mode préféré de réalisation de l'invention, la composition comprend des particules de cire de taille moyenne en volume inférieure ou égale à 1$\mu$m dispersées dans la phase aqueuse et une poudre de cire en dispersion dans la phase huileuse.

L'invention a également pour objet un procédé non thérapeutique de maquillage ou de soin de la peau comprenant l'application sur la peau d'une composition telle que définie précédemment.

L'invention a aussi pour objet l'utilisation d'une composition telle que définie précédemment pour estomper les rides et/ou donner à la peau un aspect ferme et tonique.

L'invention a encore pour objet l'utilisation d'une composition telle que définie précédemment pour obtenir un maquillage de la peau estompant les rides et/ou conférant à la peau un aspect ferme et tonique.

La taille moyenne en volume des particules peut être mesurée par différentes techniques, on peut citer en particulier les techniques de diffusion de la lumière (dynamiques et statiques), les méthodes par compteur Coulter, les mesures par vitesse de sédimentation (reliée à la taille via la loi de Stokes) et la microscopie. Ces techniques permettent de mesurer un diamètre de particules et pour certaines d'entre elles une distribution granulométrique.

De préférence, les tailles et les distributions de tailles des particules des compositions selon l'invention, sont mesurées par diffusion statique de la lumière au moyen d'un granulomètre commercial de type MasterSizer 2000 de chez Malvern. Les données sont traitées sur la base de la théorie de diffusion de Mie. Cette théorie, exacte pour des particules isotropes, permet de déterminer dans le cas de particules non sphériques, un diamètre « effectif » de particules. Cette théorie est notamment décrite dans l'ouvrage de Van de Hulst, H.C., "Light Scattering by Small Particles," Chapitres 9 et 10, Wiley, New York, 1957.

Les particules de la composition sont caractérisées par leur diamètre « effectif » moyen en volume D[4,3], défini de la manière suivante :

$$D[4,3] = \frac{\sum_i V_i \cdot d_i}{\sum_i V_i}$$

où $V_i$ représente le volume des particules de diamètre effectif $d_i$. Ce paramètre est notamment décrit dans la documentation technique du granulomètre.

Les mesures sont réalisées à 25 °C, sur une dispersion de particules diluée, obtenue à partir de la composition de la manière suivante : 1) dilution d'un facteur 100 avec le solvant majoritaire de la phase à analyser, 2) homogénéisation de la solution, 3) repos de la solution durant 18 heures, 4) récupération du surnageant homogène blanchâtre.

Le diamètre « effectif » est obtenu en prenant un indice de réfraction de 1,33 pour l'eau et un indice de réfraction moyen de 1,42 pour les particules.

Les particules de cire(s) de la phase cireuse dans les compositions selon l'invention peuvent être caractérisées par une taille exprimée en diamètre « effectif » moyen en volume D[4,3] inférieure ou égale à 1 μm, notamment inférieure ou égale à 0,75 μm et mieux inférieure ou égale à 0,55 μm.

La composition selon l'invention comprend une dispersion de particules d'un composé dans la phase aqueuse. Les particules du composé dispersées dans la phase aqueuse ont une taille moyenne en volume inférieure ou égale à 1 μm (notamment allant de 0,1 nm à 1 μm), de préférence inférieure ou égale à 0,5 μm (notamment allant de 3 nm à 0,5 μm) et plus préférentiellement allant de 3 nm à 0,3 μm.

Les composés sous forme de particules dispersées dans la phase aqueuse de l'émulsion selon la présente invention peuvent être choisis parmi les cires, les minéraux et leurs mélanges, et de préférence parmi les cires.

Selon un premier mode de réalisation, la composition comprend des particules de cire de taille moyenne en volume inférieure ou égale à 1 μm dispersées dans la phase aqueuse. Une telle dispersion est aussi appelée microdispersion aqueuse de particules de cire.

Dans la présente demande, une cire est un composé lipophile, solide à température ambiante (25 °C), à changement d'état solide/liquide réversible, ayant un point de fusion supérieur ou égal à 30 °C pouvant aller jusqu'à 120 °C.

Le point de fusion de la cire peut être mesuré à l'aide d'un calorimètre à balayage différentiel (D.S.C.), par exemple le calorimètre vendu sous la dénomination DSC 30 par la société METLER. Un échantillon de 15 mg de produit disposé dans un creuset est soumis à une première montée en température allant de 0 °C à 120 °C, à la vitesse de chauffe de 10 °C/ minute, puis est refroidi de 120 °C à 0 °C à une vitesse de refroidissement de 10 °C/minute et enfin soumis à une deuxième montée en température allant de 0 °C à 120 °C à une vitesse de chauffe de 5 °C/minute. Pendant la deuxième montée en température, on mesure la variation de la différence de puissance absorbée par le creuset vide et par le creuset contenant l'échantillon de produit en fonction de la température. Le point de fusion du composé est la valeur de la température correspondant au sommet du pic de la courbe représentant la variation de la différence de puissance absorbée en fonction de la température.

Les cires susceptibles d'être utilisées sous forme de microdispersion dans la composition selon l'invention sont choisies parmi les cires, solides et rigides, à température ambiante d'origine animale, végétale, minérale ou de synthèse et leurs mélanges. De préférence, les cires peuvent présenter un point de fusion supérieur ou égal à 45°C environ (notamment allant de 45 °C à 120 °C), et en particulier supérieur ou égal à 55°C (notamment allant de 55 °C à 120 °C), voire même supérieur ou égal à 70 °C (notamment allant de 70 °C à 120 °C).

Les cires, au sens de la demande, peuvent être hydrocarbonées, siliconées et/ou fluorées, comportant éventuellement des fonctions ester ou hydroxyle. Elles sont notamment d'origine naturelle comme la cire d'abeilles éventuellement modifiée, la cire de Carnauba, de Candellila, d'Ouricoury, du Japon, de fibres de liège ou de canne à sucre, la cérésine, les cires de paraffine, de lignite, les cires microcristallines, la cire de lanoline, la cire de Montan, les ozokérites, les huiles hydrogénées comme l'huile de jojoba hydrogénée ou copolymérisation de l'éthylène, les cires obtenues par synthèse de Fischer-Tropsch, les esters d'acides gras et les glycérides concrets à 45°C, les cires de silicone comme les alkyle, alcoxy et/ou esters de poly(di)méthylsiloxane solide à 45°C, ayant de 10 à 45 atomes de carbone, certains acides gras comme l'acide stéarique, myristique, ou béhénique, et leurs mélanges.

Les microdispersions de cire sont des dispersions de particules colloïdales de cire, et sont notamment décrites dans "Microemulsions Theory and Practice", L.M. Prince Ed., Academic Press (1977) pages 21-32.
En particulier, ces microdispersions de cire peuvent être obtenues par fusion de la cire en présence d'un tensioactif, et éventuellement d'une partie de l'eau, puis addition progressive d'eau chaude avec agitation. On observe la formation intermédiaire d'une émulsion du type eau-dans-huile, suivie d'une inversion de phase avec obtention finale d'une microémulsion du type huile-dans-eau. Au refroidissement, on obtient une microdispersion stable de particules colloïdales solides de cire. Les microdispersions de cire peuvent également être obtenues par agitation du mélange de cire, de tensioactif et d'eau à l'aide de moyen d'agitation tels que les ultrasons, l'homogénéisateur haute pression, les turbines.

Les particules de la microdispersion de cire ont de préférence des tailles moyennes en volume inférieures à 1 $\mu$m (notamment allant de 0,02 $\mu$m à 1 $\mu$m), de préférence inférieures à 0,5 $\mu$m (notamment allant de 0,05 $\mu$m à 0,5 $\mu$m) et plus préférentiellement allant de 0,05 $\mu$m à 0,3 $\mu$m.

Les particules de cires sont constituées essentiellement d'une cire ou d'un mélange de cires. Elles peuvent toutefois comprendre en proportions minoritaires des additifs gras huileux et/ou pâteux, un tensioactif et/ou un additif/ actif liposoluble usuel.

Selon un deuxième mode de réalisation, la composition comprend des particules de composés minéraux pouvant être choisis parmi la silice et les alumines (comme la boehmite), et de préférence parmi la silice, de taille moyenne en volume inférieure à 1$\mu$m, et dispersées dans la phase aqueuse.

Les particules de taille moyenne en volume inférieure ou égale à 1$\mu$m dispersées dans la phase aqueuse peuvent être présentes en une teneur allant de 0,01% à 15% en poids par rapport au poids total de la composition, de préférence de 0,5% à 10% en poids, préférentiellement de 1% à 8% en poids, et plus préférentiellement de 1 % à 7% en poids.

La phase aqueuse de l'émulsion selon l'invention peut comprendre un tensioactif dispersant permettant de disperser les particules de taille moyenne en volume inférieure ou égale à 1 $\mu$m, en particulier les particules de cire.
Le tensioactif peut être choisi parmi les composés suivants :

- les tensioactifs anioniques, notamment des sels d'acides gras éventuellement insaturés, ayant par exemple 12 à 18 atomes de carbone; des sels alcalins ou sels de bases organiques des acides alkyl-sulfuriques et alkylsulfoniques ayant 12 à 18 atomes de carbone ou d'acides alkyl-arylsulfoniques dont la chaîne alkyle contient 6-18 atomes de carbone; les éthers-sulfates.
- les tensioactifs non ioniques, notamment des tensioactifs polyalcoxylés et/ou polyglycérolés, et en particulier des acides gras ou amides d'acide gras; des alcools gras ou des alkylphénols; les esters d'acides gras et de polyols; les alcanediols et les alkyléthers d'alcanediols. On peut citer également les alkylcarbamates de triglycérol, les dérivés oxyéthylénés ou propoxylés des alcools de lanoline, des acides gras de la lanoline, ou de leur mélanges.
- les tensioactifs cationiques, notamment les dérivés d'ammonium quaternaire.

La phase aqueuse de l'émulsion selon l'invention comprend de l'eau. L'eau peut être une eau florale telle que l'eau de bleuet et/ou une eau minérale telle que l'eau de VITTEL, l'eau de LUCAS ou l'eau de LA ROCHE POSAY et/ou une eau thermale. L'eau peut être présente dans l'émulsion selon l'invention en une teneur allant de 10 % à 80 % en poids, par rapport au poids total de la composition, de préférence allant de 15 % à 60 % en poids, et préférentiellement allant de 20 % à 50 % en poids.

La phase aqueuse peut également comprendre des solvants organiques miscibles à l'eau (à température ambiante - 25 °C) comme par exemple les monoalcools ayant de 2 à 6 atomes de carbone tels que l'éthanol, l'isopropanol ; les polyols ayant notamment de 2 à 20 atomes de carbones, de préférence ayant de 2 à 10 atomes de carbone, et préférentiellement ayant de 2 à 6 atomes de carbone, tels que la glycérine, le propylène glycol, le butylène glycol, le pentylène glycol, l'hexylène glycol, le dipropylène glycol, le diéthylène glycol ; les éthers de glycol (ayant notamment de 3 à 16 atomes de carbone) tels que les alkyl($C_1$-$C_4$)éther de mono, di- ou tripropylène glycol, les alkyl($C_1$-$C_4$)éthers de mono, di- ou triéthylène glycol ; et leurs mélanges.

L'émulsion selon l'invention peut comprendre un solvant organique miscible à l'eau, notamment un polyol, en une teneur allant de 1 % à 20 % en poids, par rapport au poids total de la composition, et de préférence allant de 3 % à 15 % en poids.

La phase aqueuse peut comprendre en outre des agents de stabilisation, par exemple le chlorure de sodium, le dichlorure de magnésium et le sulfate de magnésium.

La phase aqueuse peut également comprendre tout composé hydrosoluble ou hydrodispersible compatible avec une phase aqueuse tels que des gélifiants, des polymères filmogènes, des épaississants, des tensio-actifs et leurs mélanges.

De préférence, la phase aqueuse peut être présente dans l'émulsion selon l'invention en une teneur allant 10 % à 80 % en poids, par rapport au poids total de l'émulsion, de préférence allant de 15 % à 60 % en poids, et préférentiellement allant de 20 % à 50 % en poids.

La composition selon l'invention comprend une phase huileuse contenant des particules.

Selon un mode de réalisation, les particules dispersées dans la phase huileuse ont une taille moyenne en volume inférieure ou égale à 50 $\mu$m (notamment allant de 1 à 50 $\mu$m), de préférence inférieure ou égale à 30 $\mu$m (notamment allant de 1 à 30 $\mu$m), préférentiellement inférieure ou égale à 15 $\mu$m (notamment allant de 1 à 15 $\mu$m) et plus préférentiellement inférieure ou égale à 10 micromètres.

Selon un autre mode de réalisation, les particules dispersées dans la phase huileuse ont une taille moyenne en volume inférieure ou égale à 15 $\mu$m (notamment allant de 1 à 15 $\mu$m), de préférence inférieure ou égale à 10 $\mu$m (notamment allant de 1 à 10 $\mu$m).

Selon un mode particulier de réalisation, les particules dispersées dans la phase huileuse sont choisies parmi les poudres de cire, les poudres de silice, et leurs mélanges.

Selon un aspect de l'invention, ces particules peuvent être choisies parmi poudres de cires.

Les cires présentes dans la phase huileuses sont sous forme de petites particules ayant notamment une taille moyenne en volume allant de 0,5 à 30 micromètres, en particulier allant de 1 à 20 micromètres, et plus particulièrement allant de 5 à 10 micromètres, désignées par la suite par l'expression « micro cires ».

Comme micro cires pouvant être utilisées dans les compositions selon l'invention, on peut citer notamment les micro cires de carnauba telles que celle commercialisée sous la dénomination de « MicroCare 350® » par la société MICRO POWDERS, les micro cires de cire synthétique telles que celle commercialisée sous la dénomination de « MicroEase 114S® » par la société MICRO POWDERS, les micro cires constituées d'un mélange de cire de carnauba et de cire de polyéthylène telles que celles commercialisées sous les dénominations de « Micro Care 300®» et « 310® » par la société MICRO POWDERS, les micro cires constituées d'un mélange de cire de carnauba et de cire synthétique telles que celle commercialisée sous la dénomination « Micro Care 325® » par la société MICRO POWDERS, les micro cires de polyéthylène telles que celles commercialisées sous les dénominations de « Micropoly 200® », « 220® », « 220L® » et « 250S® » par la société MICRO POWDERS.

Selon un autre mode de réalisation, les particules dispersées dans la phase huileuse peuvent être des poudres de silice.

Les poudres de silice peuvent être par exemple les microsphères de silice poreuses vendues sous la dénomination "SILICA BEADS SB-700" commercialisées par la société MYOSHI, les "SUNSPHERE® H51", "SUNSPHERE® H33" , "SUNSPHERE® H53" commercialisées par la société ASAHI GLASS ; les microsphères de silice amorphe enrobées de polydiméthylsiloxane vendues sous la dénomination "SA SUNSPHERE® H-33" et "SA SUNSPHERE® H-53" commercialisées par la société ASAHI GLASS ; les silices pyrogénées hydrophobes, notamment les silices ayant des groupements hydrophobes triméthylsiloxyl par exemple commercialisées sous les références "AEROSIL R812®" par la société Degussa, "CAB-O-SIL TS-530®" par la société Cabot, ou les silices ayant des groupements hydrophobes diméthylsilyloxyl ou polydiméthylsiloxane, par exemple commercialisées sous les références "AEROSIL R972®", "AEROSIL R974®" par la société Degussa, "CAB-O-SIL TS-610®", "CAB-O-SIL TS-720®" par la société Cabot.

Les particules dispersées dans la phase huileuse de l'émulsion selon l'invention peuvent être une charge « soft-focus » telle que décrite dans EP-A-1099437.

Les dites particules de taille inférieure à 50$\mu$m dispersées dans la phase huileuse peuvent être présentes dans la composition selon l'invention en une teneur allant de 0,1% à 15% en poids par rapport au poids total de la composition, de préférence allant de 0,5% à 10% en poids, et plus préférentiellement, allant de 0,5% à 8% en poids.

L'émulsion selon l'invention peut contenir, dans sa phase huileuse, de 0,1% à 15% en poids de cires par rapport au poids total de la composition. En particulier, elle peut contenir de 0,5% à 10%, et plus particulièrement de 0,5% à 8% en poids.

La phase huileuse de la composition selon l'invention comprend au moins une huile pouvant être choisie parmi les huiles volatiles, les huiles non volatiles, et leurs mélanges. Avantageusement, la composition comprend au moins une huile volatile et au moins une huile non volatile.

La composition selon l'invention peut comprendre au moins une huile volatile.

Par « huile volatile », on entend au sens de l'invention toute huile susceptible de s'évaporer au contact de la peau, à température ambiante et pression atmosphérique. Les huiles volatiles de l'invention sont des huiles cosmétiques volatiles, liquides à température ambiante, ayant une pression de vapeur non nulle, à température ambiante et pression atmosphérique, allant en particulier de 0,13 Pa à 40.000 Pa (0,001 à 300 mm de Hg) et de préférence allant de 1,3 à 1300 Pa (0,01 à 10 mm de Hg).

L'huile volatile peut être choisie parmi les huiles volatiles hydrocarbonées, les huiles volatiles siliconées, les huiles volatiles fluorées, et leurs mélanges.

On entend par "huile hydrocarbonée", une huile contenant principalement des atomes d'hydrogène et de carbone et éventuellement des atomes d'oxygène, d'azote, de soufre et/ou de phosphore.

Les huiles hydrocarbonées volatiles peuvent être choisies parmi les huiles hydrocarbonées ayant de 8 à 16 atomes de carbone, et notamment les alcanes ramifiés en $C_8$-$C_{16}$ comme les isoalcanes en $C_8$-$C_{16}$ d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars® ou de Permethyls®.

Comme huiles volatiles, on peut aussi utiliser les silicones volatiles, comme par exemple les huiles de silicones linéaires ou cycliques volatiles, notamment celles ayant une viscosité ≤ 5 centistokes (5 x $10^{-6}$ $m^2$/s), et ayant notamment de 2 à 10 atomes de silicium, de préférence de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

L'huile volatile fluorée n'a généralement pas de point éclair.

Comme huile volatile fluorée, on peut citer le nonafluoroéthoxybutane, le nonafluorométhoxybutane, le décafluoropentane, le tétradécafluorohexane, le dodécafluoropentane, et leurs mélanges.

Avantageusement, la composition comprend au moins une huile volatile hydrocarbonée, et notamment un mélange d'isododécane et d'isohexadécane.

L'huile volatile peut être présente dans la composition selon l'invention en une teneur allant de 1 % à 60 % en poids, par rapport au poids total de la composition, de préférence allant de 10 % à 55 % en poids, préférentiellement allant de 15 % à 50 % en poids, et plus préférentiellement allant de 20 % à 50 % en poids.

La composition selon l'invention peut comprendre au moins une huile non volatile.

Par "huile non volatile", on entend une huile restant sur la peau à température ambiante et pression atmosphérique au moins plusieurs heures et ayant notamment une pression de vapeur inférieure à 0,13 Pa (0,01 mm de Hg).

Ces huiles non volatiles peuvent être des huiles hydrocarbonées notamment d'origine animale ou végétale, des huiles siliconées, ou leurs mélanges. On entend par "huile hydrocarbonée", une huile contenant principalement des atomes d'hydrogène et de carbone et éventuellement des atomes d'oxygène, d'azote, de soufre et/ou de phosphore.

Les huiles non volatiles peuvent notamment être choisies parmi les huiles hydrocarbonées le cas échéant fluorées et/ou les huiles siliconées non volatiles.

Comme huile hydrocarbonée non volatile, on peut notamment citer :

- les huiles hydrocarbonées d'origine animale,

- les huiles hydrocarbonées d'origine végétale telles que les triglycérides constitués d'esters d'acides gras et de glycérol dont les acides gras peuvent avoir des longueurs de chaînes variées de $C_4$ à $C_{24}$, ces dernières pouvant être linéaires ou ramifiées, saturées ou insaturées ; ces huiles sont notamment des triglycérides d'acide heptanoïque ou d'acide octanoïque, ou bien encore les huiles de germe de blé, de tournesol, de pépins de raisin, de sésame, de maïs, d'abricot, de ricin, de karité, d'avocat, d'olive, de soja, d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; le beurre de karité ; ou encore les triglycérides des acides caprylique/caprique comme ceux vendus par la société Stéarineries Dubois ou ceux vendus sous les dénominations Miglyol 810®, 812® et 818® par la société Dynamit Nobel,

- les éthers de synthèse ayant de 10 à 40 atomes de carbone ;

- les hydrocarbures linéaires ou ramifiés, d'origine minérale ou synthétique tels que la vaseline, les polydécènes, le polyisobutène hydrogéné tel que le Parleam ®, le squalane, les huiles de paraffine, et leurs mélanges,

- les esters de synthèse comme les huiles de formule $R_1COOR_2$ dans laquelle $R_1$ représente le reste d'un acide gras linéaire ou ramifié comportant de 1 à 40 atomes de carbone et $R_2$ représente une chaîne hydrocarbonée notamment ramifiée contenant de 1 à 40 atomes de carbone à condition que $R_1$ + $R_2$ soit ≥ 10, comme par exemple l'huile de Purcellin (octanoate de cétostéaryle), le myristate d'isopropyle, le palmitate d'isopropyle, les benzoates d'alcools en $C_{12}$ à $C_{15}$, le laurate d'hexyle, l'adipate de diisopropyle, l'isononanoate d'isononyle, le néopentanoate d'isodecyl, le palmitate de 2-éthyl-hexyle, l'isostéarate d'isostéaryle, le laurate de 2-hexyl-décyle, le palmitate de 2-octyl-décyle, le myristate de 2-octyl-dodécyle, des heptanoates, octanoates, décanoates ou ricinoléates d'alcools ou de polyalcools comme le dioctanoate de propylène glycol ; les esters hydroxylés comme le lactate d'isostéaryle, le malate de di-isostéaryle, le lactate de 2-octyl-dodécyle ; les esters de polyols et les esters du pentaérythritol,

- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, et le 2-undécylpentadécanol,

- les acides gras supérieurs tels que l'acide oléique, l'acide linoléique, l'acide linolénique et leurs mélanges.

Les huiles de silicone non volatiles utilisables dans la composition selon l'invention peuvent être les polydiméthylsiloxanes (PDMS) non volatiles, les polydiméthylsiloxanes comportant des groupements alkyle ou alcoxy pendants et/ou en bouts de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, et leurs mélanges.

Avantageusement, l'huile non volatile peut être choisie parmi les esters en $C_{12}$-$C_{36}$ tels que ceux décrits précédemment.

L'huile non volatile peut être présente dans la composition selon l'invention en une teneur allant de 1 % à 60 % en poids, par rapport au poids total de la composition, de préférence allant de 5 % à 50 % en poids, de préférence allant de 5 % à 40 % en poids.

La phase huileuse de l'émulsion peut être présente en une teneur allant de 1 % à 60 % en poids, par rapport au poids total de la composition, de préférence allant de 5 % à 50 % en poids, de préférence allant de 5 % à 40 % en poids.

L'émulsion selon l'invention peut comprendre un tensioactif ou un mélange de tensioactifs, notamment un tensioactif dont le HLB (balance hydrophile/lipophile) est adapté au sens de l'émulsion.

Comme tensioactif utilisable dans l'invention, adapté à l'obtention d'une émulsion E/H on peut citer ceux ayant un HLB inférieur 7 et notamment les ester d'acides gras de polyols comme les mono-, di-, tri- ou sesqui-oléates ou stéarates de sorbitol ou de glycérol, les laurates de glycérol ou de polyéthylène glycol ; les alkyl ou alkoxy diméthicones copolyols à chaîne alkyle ou alkoxy pendante ou en bout de squelette siliconé ayant par exemple de 6 à 22 atomes de carbone.

Comme tensioactif utilisable dans l'invention pour l'obtention d'une émulsion H/E on peut citer ceux ayant un HLB supérieur à 7 comme les esters d'acides gras de polyéthylène glycol (monostéarate ou monolaurate de polyéthylène glycol) ; les esters d'acides gras (stéarate, oléate) de sorbitol polyoxyéthylénés ; les alkyl (lauryl, cétyl, stéaryl, octyl) éthers polyoxyéthylénés et les diméthicones copolyols. De façon générale, on peut utiliser tout tensioactif ionique (cationique ou anionique) amphotère et tout tensioactif non ionique, bien connu de l'homme du métier.

Le tensioactif peut être présent dans la composition en une teneur allant de 0,3 % à 10 % en poids, par rapport au poids total de la composition, et de préférence allant de 1 % à 5 % en poids.

L'émulsion selon l'invention peut comprendre en outre des charges additionnelles différentes des particules décrites précédemment.

Par charges, il faut comprendre des particules de toute forme, incolores ou blanches, minérales ou de synthèse, insolubles dans le milieu de la composition quelle que soit la température à laquelle la composition est fabriquée.

Les charges peuvent être minérales ou organiques de toute forme, plaquettaires, sphériques ou oblongues, quelle que soit la forme cristallographique (par exemple feuillet, cubique, hexagonale, orthorombique, etc.). On peut citer le talc, le mica, la silice, le kaolin, les poudres de polyamide (Nylon®), de poly-β-alanine et de polyéthylène, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique, les microbilles de résine de silicone (Tospearls® de Toshiba, par exemple), les particules de polyorganosiloxanes élastomères, le carbonate de calcium précipité, le carbonate et l'hydro-carbonate de magnésium, l'hydroxyapatite, les microsphères de silice creuses, les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium, les particules de polyméthacrylate de méthyle.

Les charges additionnelles peuvent être présentes dans la composition en une teneur allant de 0,1 % à 25 % en poids, par rapport au poids total de la composition, de préférence allant de 1 % à 20 % en poids, et préférentiellement allant de 5 % à 15 % en poids.

L'émulsion selon l'invention peut comprendre au moins une matière colorante pouvant être choisie parmi les colorants hydrosolubles ou liposolubles, les pigments, les nacres et leurs mélanges.

Par « pigments », il faut comprendre des particules blanches ou colorées, minérales ou organiques, insolubles dans la phase organique liquide, destinées à colorer et/ou opacifier la composition.

Les pigments et nacres sont des particules à l'état solide au sein de la composition finale. Ces particules solides ne sont pas solubilisées dans la composition finale. Par « nacres », il faut comprendre des particules irisées, notamment produites par certains mollusques dans leur coquille ou bien synthétisées, insolubles dans le milieu de la composition. Par « colorants », il faut comprendre des composés généralement organiques solubles dans les corps gras comme les huiles ou dans une phase hydroalcoolique.

Les pigments peuvent être des pigments minéraux ou organiques. Comme pigments, on peut utiliser les oxydes métalliques comme les oxydes de fer (notamment ceux de couleur jaune, rouge, brun, noir), les dioxydes de titane, l'oxyde de cérium, l'oxyde de zirconium, l'oxyde de chrome ; le violet de manganèse, l'ultramarine bleue, le bleu de prusse, le bleu outremer, le bleu ferrique, l'oxychlorure de bismuth, la nacre, le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth, et leurs mélanges.

On utilise de préférence des pigments d'oxydes de fer ou de dioxyde de titane.

Les pigments peuvent être traités avec un agent hydrophobe pour les rendre compatible avec la phase organique de la composition. L'agent de traitement hydrophobe peut être choisi parmi les silicones comme les méthicones, les diméthicones, les perfluoroalkylsilanes ; les acides gras comme l'acide stéarique ; les savons métalliques comme le dimyristate d'aluminium, le sel d'aluminium du glutamate de suif hydrogéné, les perfluoroalkyl phosphates, les perfluoroalkyl si lanes, les perfluoroalkyl silazanes, les polyoxydes d'hexafluoropropylène, les polyorganosiloxanes comprenant des groupes perfluoroalkylles perfluoropolyéthers, les acides aminés ; les acides aminés N-acylés ou leurs sels ; la lécithine, le trisostéaryle titanate d'isopropyle, et leurs mélanges.

Les acides aminés N-acylés peuvent comprendre un groupe acyle ayant de 8 à 22 atomes de carbones, comme par exemple un groupe 2-éthyl hexanoyle, caproyle, lauroyle, myristoyle, palmitoyle, stéaroyle, cocoyle. Les sels de ces composés peuvent être les sels d'aluminium, de magnésium, de calcium, de zirconium, de zin, de sodium, de potassium.

L'acide aminé peut être par exemple la lysine, l'acide glutamique, l'alanine

Le terme alkyl mentionné dans les composés cités précédemment désigne notamment un groupe alkyle ayant de 1 à 30 atomes de carbone, de préférence ayant de 5 à 16 atomes de carbone.

Des pigments traités hydrophobes sont notamment décrits dans la demande EP-A-1086683.

Les colorants liposolubles sont par exemple le rouge Soudan, le D&C Red n° 17, le D&C Green n° 6, le β-carotène, l'huile de soja, le brun Soudan, le D&C Yellow n° 11, le D&C Violet n° 2, le D&C orange n° 5, le jaune quinoléine, le rocou, les bromoacides.

Les colorants hydrosolubles sont par exemple le jus de betterave, le bleu de méthylène, le caramel.

Les matières colorantes peuvent être présentes dans la composition en une teneur allant de 0,001 % à 30 % en poids, par rapport au poids total de la composition, de préférence allant de 0,1 % à 20 % en poids, et préférentiellement allant de 1 à 15 % en poids.

La composition peut se présenter sous forme d'émulsion eau-dans-huile, huile-dans-eau, d'émulsion multiple.

Selon un mode préféré, la composition se présente sous forme d'émulsion eau-dans-huile, huile-dans-eau.

La composition selon l'invention peut comprendre au moins un autre ingrédient cosmétique usuel pouvant être choisi notamment parmi les agents gélifiants et/ou épaississants hydrophiles ou lipophiles, les antioxydants, les parfums, les conservateurs, les neutralisants, les filtres solaires, les vitamines, les hydratants, les composés auto-bronzants, les actifs antirides, les émollients, les actifs hydrophiles ou lipophiles, les agents anti-radicaux libres, les sequestrants, les agents filmogènes, et leurs mélanges.

L'invention est illustrée plus en détails par les exemples décrits ci-après.

Exemple 1

On a préparé un fond de teint sous forme d'émulsion eau-dans-huile ayant la composition suivante :

| | |
|---|---|
| Silice vendue sous la dénomination SUNSPHERE H 51 par la société Asahi Glass | 4g |
| Cétyl diméthicone copolyol (ABIL EM 90 par la société GOLDSCHMIDT) | 2,7g |
| Isostéarate de polyglycéryle-4 (Isolan GI 34 par la société GOLDSCHMIDT) | 0,9g |
| Palmitate d'isostéaryle | 6,0g |
| Cyclopentasiloxane | 30 g |
| Oxydes de fer enrobés hydrophobes | 3,13g |
| Oxydes de titane enrobés hydrophobes | 7,87g |

(suite)

| | |
|---|---|
| Dispersion aqueuse de silice colloïdale (40% MA) vendue sous la Dénomination COSMO S 40 par la société Catalysts & Chemicals | 17g |
| Eau | qsp 100g |

On mélange les huiles. On broie les pigments avec de la cyclopentasiloxane. On ajoute le broyat dans le mélange d'huiles en agitant à température ambiante.
On disperse ensuite la silice par agitation.
Puis on ajoute la dispersion aqueuse de silice et l'eau en refroidissant de manière à conserver une température d'émulsification proche de l'ambiante.
L'émulsion obtenue contient une dispersion aqueuse de silice colloïdale et de la silice dispersée dans la phase huileuse.
Le fond de teint appliqué sur la peau permet d'obtenir un maquillage qui masque efficacement les rides et donne à la peau un aspect ferme et tonique.

Exemple 2

On prépare un fond de teint sous forme d'émulsion eau-dans-huile ayant la composition suivante :

| | |
|---|---|
| Poudre de cire de paraffine vendue sous la dénomination MICROEASE 114S par la société Micropowders | 4,0g |
| Cétyl diméthicone copolyol (ABIL EM 90 par la société GOLDSCHMIDT) | 2,7g |
| Isostéarate de polyglycéryle-4 (Isolan GI 34 par la société GOLDSCHMIDT) | 0,9g |
| Palmitate d'isostéaryle | 6,0g |
| Cyclopentasiloxane | 20 g |
| Oxydes de fer enrobés hydrophobes | 3,13g |
| Oxydes de titane enrobés hydrophobes | 7,87g |
| Dispersion aqueuse de silice colloïdale (40% MA) vendue sous la Dénomination COSMO S 40 par la société Catalysts & Chemicals | 17g |
| Eau | qsp 100g |

Le mode de préparation est le même que celui de l'exemple 1.
L'émulsion obtenue contient une dispersion aqueuse de silice colloïdale et de la poudre de cire dans la phase huileuse.
Le fond de teint appliqué sur la peau permet d'obtenir un maquillage qui masque efficacement les rides et donne à la peau un aspect ferme et tonique.

Exemple 3 :

On a préparé, dans un premier temps, une microdispersion à 27% MA de cire de carnauba ayant la composition suivante :

| | |
|---|---|
| - Cire de carnauba | 27 g |
| - Monostéarate de glycéryle polyoxyéthyléné (30 OE) (TAGAT S de GOLDSCHMIDT) | 6,75 g |
| - Ethanol | 10 g |
| - Eau                              qsp | 100 g |

On a chauffé à 90 °C la cire et le tensioactif en homogénéisant le mélange sous agitation modérée. Puis on a incorporé l'eau chauffée à 90 °C en continuant d'agiter. On a refroidi à température ambiante et ajouté l'éthanol pour obtenir une microdispersion de cire ayant un diamètre moyen de particules d'environ 170 nm.
On a préparé ensuite un fond de teint sous forme d'émulsion huile-dans-eau comprenant la micro dispersion de cire précédemment préparée, ayant la composition suivante :

| | |
|---|---|
| Polydiméthylesiloxane (Dow Corning fluid 200 5 cstde la société Dow Corning) | 9,00g |

(suite)

| | |
|---|---|
| Triglycéryl d'acides caprylique/caprique (MYRITOL de la société COGNIS) | 10,45g |
| Néopentanoate d'isodécyle | 6,0g |
| Micro sphères de silice (SUNSPHERE H51 de la société Dohkaï Chemical Industries) | 4,0g |
| Poudre de cire de paraffine (Microease 114S de la société Micro Powders) | 1,0g |
| Micro dispersion de cire de carnauba dans l'eau à 27% MA de cire | 10g |
| Diméthicone copolyol (Abil Care 85 de la société GOLDSCHMIDT) | 3,45g |
| Gélifiant (SEPIGEL 305 de la société Seppic) | 0,20g |
| Glycérol | 5,0g |
| Butylène glycol | 4,0g |
| Mono oléate de sorbitane oxyéthylène (TWEEN 80V PHARMA de la société UNIQUEMA) | 10g |
| Silicate d'aluminium et de magnésium (Veegum granules de la société Vanderbilt) | 0,50g |
| Talc | 3,00g |
| Dioxyde de Titane | 8,75g |
| Oxyde de fer | 2,25g |
| Conservateurs | qs |
| Eau | qsp 100g |

On introduit dans l'eau les conservateurs et les agents hydratants. On y ajoute, à température ambiante, la micro dispersion de cire préalablement réalisée. On broie les pigments dans un mélange eau- Silicate d'aluminium et de magnésium, puis on ajoute le broyat à la phase aqueuse.

On mélange les huiles, les tensioactifs et les microcires.

On réalise l'émulsion à température ambiante puis on ajoute le talc, les micro sphères de silice et le gélifiant.

L'émulsion obtenue contient une dispersion aqueuse de cires et de la poudre de cire dans la phase huileuse.

Le fond de teint appliqué sur la peau permet d'obtenir un maquillage qui masque efficacement les rides et donne à la peau un aspect ferme et tonique.

## Revendications

1. Composition cosmétique de maquillage ou de soin de la peau sous forme d'émulsion comprenant une phase aqueuse et une phase huileuse, **caractérisée en ce qu'**elle comprend une dispersion de particules de cire de taille moyenne en volume inférieure ou égale à 1 $\mu$m dans la phase aqueuse et des particules de taille moyenne en volume inférieure ou égale à 15 $\mu$m dispersée dans la phase huileuse.

2. Composition cosmétique de maquillage ou de soin de la peau sous forme d'émulsion comprenant une phase aqueuse et une phase huileuse, **caractérisée en ce qu'**elle comprend une dispersion d'un composé sous forme de particules de taille moyenne en volume inférieure ou égale à 1$\mu$m dans la phase aqueuse et une dispersion de particules du même composé de taille moyenne en volume inférieure ou égale à 50 $\mu$m dans la phase huileuse.

3. Composition selon la revendication précédente **caractérisée en ce que** les particules de taille moyenne en volume inférieure ou égale à 1 $\mu$m dispersées dans la phase aqueuse sont choisies parmi les cires, les composés minéraux et leurs mélanges.

4. Composition selon la revendication précédente, **caractérisée en ce que** les composés minéraux sont choisis parmi les silices, les alumines, ou leurs mélanges.

5. Composition cosmétique de maquillage ou de soin de la peau sous forme d'émulsion comprenant une phase aqueuse et une phase huileuse, **caractérisée en ce qu'**elle comprend des particules de taille moyenne en volume inférieure ou égale à 1 $\mu$m dispersées dans la phase aqueuse choisies parmi les cires, les particules de silice, et leurs mélanges, et des particules dispersées dans la phase huileuse choisies parmi les poudres de cire, les poudres de silice, et leurs mélanges.

6. Composition cosmétique selon la revendication précédente **caractérisée en ce qu'**elle comprend des particules de cire de taille moyenne en volume inférieure ou égale à 1$\mu$m dispersées dans la phase aqueuse et une poudre de cire en dispersion dans la phase huileuse.

7. Composition selon l'une quelconque des revendications 1 et 3 à 6, **caractérisée en ce que** la dispersion de cires dans la phase aqueuse comprend des particules de cires ayant une taille moyenne en volume inférieure ou égale à 0,5 μm.

8. Composition selon l'une quelconque des revendications 1 et 3 à 7, **caractérisée en ce que** la cire est choisie dans le groupe formé par la cire de Carnauba, de Candellila, d'Ouricoury, du Japon, de fibres de liège ou de canne à sucre, la cérésine, les cires de paraffine, de lignite, les cires microcristallines, la cire de lanoline, la cire de Montan, les ozokérites, les huiles hydrogénées comme l'huile de jojoba hydrogénée ou copolymérisation de l'éthylène, les cires obtenues par synthèse de Fischer-Tropsch, les esters d'acides gras et les glycérides concrets à 45°C, les cires de silicone comme les alkyle, alcoxy et/ou esters de poly(di)méthylsiloxane solide à 45°C, ayant de 10 à 45 atomes de carbone, certains acides gras comme l'acide stéarique, myristique, ou béhénique, et leurs mélanges.

9. Composition selon l'une quelconque des revendications 1 et 3 à 8, **caractérisée en ce que** la cire dispersée dans la phase aqueuse est présente en une teneur allant de 0.01% à 15% en poids par rapport au poids total de la composition, de préférence allant de 0,5% à 10% en poids, et préférentiellement, allant de 1% à 8%, et plus préférentiellement allant de 1% à 7% en poids.

10. Composition cosmétique selon l'une quelconque des revendications 2 à 9, **caractérisée en ce que** les particules dispersées dans la phase huileuse ont une taille moyenne en volume inférieure ou égale à 30 micromètres, de préférence inférieure ou égale à 15 micromètres, et plus préférentiellement inférieure ou égale à 10 micromètres.

11. Composition selon l'une quelconque des revendications précédentes, **caractérisée en ce que** lesdites particules dispersées dans la phase huileuse sont présentes en une teneur allant de 0,1 % à 15% en poids par rapport au poids total de la composition, de préférence allant de 0,5% à 10% en poids, et plus préférentiellement allant de 0,5 % à 8% en poids.

12. Composition selon l'une quelconque des revendications précédentes, **caractérisée en ce qu'**elle comprend au moins une huile.

13. Composition selon l'une quelconque des revendications précédentes, **caractérisée en ce qu'**elle comprend au moins une huile volatile.

14. Composition selon la revendication précédente, **caractérisée en ce que** l'huile volatile est choisie parmi les huiles volatiles hydrocarbonées ayant de 8 à 16 atomes de carbone; les huiles de silicones linéaires ou cycliques ayant de 2 à 7 atomes de silicium, ces silicones comportant éventuellement des groupes alkyle ou alkoxy ayant de 1 à 10 atomes de carbone ; les huiles volatiles fluorées.

15. Composition selon la revendication 13 ou 14, **caractérisée en ce que** l'huile volatile est choisie parmi l'isododécane, l'isodécane, l'isohexadécane, l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane.

16. Composition selon l'une quelconque des revendications 13 à 15, **caractérisée en ce que** l'huile volatile est présente en une teneur allant de 1 % à 60% en poids, par rapport au poids total de la composition, de préférence allant de 10% à 55% en poids, préférentiellement allant de 15 % à 50 % en poids et plus préférentiellement allant de 20% à 50%.

17. Composition selon l'une quelconque des revendications précédentes, **caractérisée en ce qu'**elle comprend une huile non volatile.

18. Composition selon la revendication précédente, **caractérisée en ce que** l'huile non volatile est choisie parmi les huiles siliconées non volatiles, les huiles hydrocarbonées non volatiles, les huiles non volatiles fluorées, et leurs mélanges.

19. Composition selon la revendication 17 ou 18, **caractérisée en ce que** l'huile non volatile est une huile non volatile siliconée,

**20.** Composition selon l'une quelconque des revendications 17 à 19, **caractérisée en ce que** l'huile non volatile est choisie parmi les polydiméthylsiloxanes non volatils ; les polydiméthylsiloxanes comportant des groupements alkyle, alcoxy ou phényle, pendant ou en bout de chaîne siliconée, groupements ayant de 2 à 24 atomes de carbone ; les silicones phénylées ; les polysiloxanes modifiés par des acides gras (notamment en C8-C20), des alcools gras (notamment en C8-C20) ou des polyoxyalkylènes (notamment polyoxyéthylène et/ou polyoxypropylène) ; les silicones aminées ; les silicones à groupement hydroxyles ; les silicones fluorés comportant un groupement fluoré pendant ou en bout de chaîne siliconée ayant de 1 à 12 atomes de carbone dont tout ou partie des hydrogène sont substitués par des atomes de fluor ; et leurs mélanges.

**21.** Composition selon l'une quelconque des revendications 17 à 20, **caractérisée en ce que** l'huile non volatile est choisie parmi l'huile de paraffine, le squalane, le polyisobutylène hydrogéné (huile de Parléam), le perhydrosqualène, l'huile de vison, de tortue, de soja, l'huile d'amande douce, de calophylium, de palme, de pépins de raisin, de sésame, de maïs, d'arara, de colza, de tournesol, de coton, d'abricot, de ricin, d'avocat, de jojoba, d'olive ou de germes de céréales ; les esters gras en $C_{12}$-$C_{36}$ ; les acides gras supérieurs en en $C_{14}$-$C_{22}$ ; les alcools gras supérieurs en $C_{16}$-$C_{22}$ ; et leurs mélanges.

**22.** Composition selon l'une quelconque des revendications 17 à 21, en ce que l'huile non volatile est présente en une teneur allant de 1 % à 60 % en poids, par rapport au poids total de la composition, et de préférence allant de 5 % à 50 % en poids.

**23.** Composition selon l'une quelconque des revendications précédentes, **caractérisée en ce qu'**elle comprend de l'eau en une teneur allant de 10% à 80% en poids par rapport au poids total de la composition, de préférence de 15% à 60% en poids, plus préférentiellement de 20% à 50% en poids.

**24.** Composition selon l'une quelconque des revendications précédentes, **caractérisée en ce que** la phase aqueuse comprend un solvant choisi parmi l'éthanol, l'isopropanol, le glycérol, le propylène glycol, le butylène glycol, l'hexylène glycol, le dipropylène glycol, le diéthylène glycol, les alkyl($C_1$-$C_4$) éthers de mono, di- ou tripropylène glycol, les alkyl($C_1$-$C_4$) éthers de mono, di- ou triéthylène glycol, et leurs mélanges.

**25.** Composition selon l'une quelconque des revendications précédentes, **caractérisée en ce que** la phase aqueuse est présente en une teneur allant de de 10% à 80% en poids par rapport au poids total de la composition, de préférence de 15% à 60% en poids, plus préférentiellement de 20% à 50% en poids.

**26.** Composition selon l'une quelconque des revendications précédentes, **caractérisée en ce qu'**elle comprend un tensioactif.

**27.** Composition selon l'une quelconque des revendications précédentes, **caractérisée en ce que** la composition se présente sous forme d'une émulsion huile-dans-eau ou eau-dans-huile.

**28.** Composition selon l'une quelconque des revendications précédentes, **caractérisée en ce qu'**elle comprend des charges additionnelles différentes desdites particules dispersées dans la phase aqueuse et dans la phase huileuse.

**29.** Composition selon la revendication précédente, **caractérisée en ce que** les charges additionnelles sont choisies parmi le talc, le mica, la silice, le kaolin, les poudres de polyamide, les poudres de poly-β-alanine, les poudres de polyéthylène, les poudres de polymères de tétrafluoroéthylène, la lauroyl-lysine, l'amidon, le nitrure de bore, les microsphères creuses de chlorure de polyvinylidène/acrylonitrile, les microsphères creuses de copolymères d'acide acrylique, les microbilles de résine de silicone, les particules de polyorganosiloxanes élastomères, le carbonate de calcium précipité, le carbonate de magnésium, l'hydro-carbonate de magnésium, l'hydroxyapatite, les microsphères de silice creuses, les silices poreuses, les microcapsules de céramique, les savons métalliques dérivés d'acides organiques carboxyliques ayant de 8 à 22 atomes de carbone.

**30.** Composition selon la revendication 28 ou 29, **caractérisée en ce que** les charges additionnelles sont présentes en une teneur allant de 0,1 % à 25% en poids, par rapport au poids total de la composition, de préférence allant de 1 % à 20% en poids et plus préférentiellement allant de 5% à 15% en poids.

**31.** Composition selon l'une quelconque des revendications précédentes, **caractérisée en ce qu'**elle comprend une matière colorante.

**32.** Composition selon la revendication précédente, **caractérisée en ce que** la matière colorante est choisie parmi les pigments, les nacres, les colorants, et leurs mélanges.

**33.** Composition selon l'une quelconque des revendications précédentes, **caractérisée en ce que** la matière colorante est présente en une teneur allant de 0,001 % à 30 % en poids, par rapport au poids total de la composition, de préférence allant de 0,1 % à 20 % en poids, et préférentiellement allant de 1 à 15 % en poids.

**34.** Composition selon l'une quelconque des revendications précédentes, **caractérisée en ce qu'**elle comprend un ingrédient cosmétique choisi parmi les agents gélifiants et/ou épaississants hydrophiles ou lipophiles, les antioxydants, les parfums, les conservateurs, les neutralisants, les filtres solaires, les vitamines, les hydratants, les composés auto-bronzants, les actifs antirides, les émollients, les actifs hydrophiles ou lipophiles, les agents anti-radicaux libres, les sequestrants, les agents filmogènes, et leurs mélanges.

**35.** Procédé non thérapeutique de maquillage ou de soin de la peau comprenant l'application sur la peau d'une composition cosmétique selon l'une quelconque des revendications précédentes.

**36.** Utilisation d'une composition selon les revendications 1 à 34 pour estomper les rides et/ou donner à la peau un aspect ferme et tonique,

**37.** Utilisation d'une composition selon les revendications 1 à 34 pour obtenir un maquillage de la peau estompant les rides et/ou conférant à la peau un aspect ferme et tonique.

**Claims**

**1.** Cosmetic composition for making up or caring for the skin in the form of an emulsion comprising an aqueous phase and an oily phase, **characterized in that** it comprises a dispersion of wax particles with a mean size by volume of less than or equal to 1 $\mu$m in the aqueous phase and, dispersed in the oily phase, particles with a mean size by volume of less than or equal to 15 $\mu$m.

**2.** Cosmetic composition for making up or caring for the skin in the form of an emulsion comprising an aqueous phase and an oily phase, **characterized in that** it comprises a dispersion of a compound in the form of particles with a mean size by volume of less than or equal to 1 $\mu$m in the aqueous phase and a dispersion of particles of the same compound with a mean size by volume of less than or equal to 50 $\mu$m in the oily phase.

**3.** Composition according to the preceding claim, **characterized in that** the particles with a mean size by volume of less than or equal to 1 $\mu$m dispersed in the aqueous phase are chosen from waxes, inorganic compounds and their mixtures.

**4.** Composition according to the preceding claim, **characterized in that** the inorganic compounds are chosen from silicas, aluminas or their mixtures.

**5.** Cosmetic composition for making up or caring for the skin in the form of an emulsion comprising an aqueous phase and an oily phase,
**characterized in that** it comprises particles with a mean size by volume of less than or equal to 1 $\mu$m dispersed in the aqueous phase chosen from waxes, silica particles and their mixtures and particles dispersed in the oily phase chosen from wax powders, silica powders and their mixtures.

**6.** Cosmetic composition according to the preceding claim, **characterized in that** it comprises wax particles with a mean size by volume of less than or equal to 1 $\mu$m dispersed in the aqueous phase and a wax powder in dispersion in the oily phase.

**7.** Composition according to any one of Claims 1 and 3 to 6, **characterized in that** the dispersion of waxes in the aqueous phase comprises particles of waxes having a mean size by volume of less than or equal to 0.5 $\mu$m.

**8.** Composition according to any one of Claims 1 and 3 to 7, **characterized in that** the wax is chosen from the group formed by carnauba, candelilla, ouricury, Japan, cork fibre or sugarcane wax, ceresin, paraffin or lignite waxes, microcrystalline waxes, lanolin wax, montan wax, ozokerites, hydrogenated oils, such as hydrogenated jojoba oil or copolymerization of ethylene, the waxes obtained by the Fischer-Tropsch synthesis, fatty acid esters and glycerides which are solid at 45°C, silicone waxes, such as the alkyl, alkoxy and/or ester derivatives of poly(di)methylsiloxane which are solid at 45°C, having from 10 to 45 carbon atoms, some fatty acids, such as stearic, myristic or behenic acid, and their mixtures.

9. Composition according to any one of Claims 1 and 3 to 8, **characterized in that** the wax dispersed in the aqueous phase is present in a content ranging from 0.01% to 15% by weight with respect to the total weight of the composition, preferably ranging from 0.5% to 10% by weight, preferentially ranging from 1% to 8% by weight and more preferentially ranging from 1% to 7% by weight.

10. Cosmetic composition according to any one of Claims 2 to 9, **characterized in that** the particles dispersed in the oily phase have a mean size by volume of less than or equal to 30 micrometres, preferably of less than or equal to 15 micrometres and more preferably of less than or equal to 10 micrometres.

11. Composition according to any one of the preceding claims, **characterized in that** the said particles dispersed in the oily phase are present in a content ranging from 0.1% to 15% by weight with respect to the total weight of the composition, preferably ranging from 0.5% to 10% by weight and more preferably ranging from 0.5% to 8% by weight.

12. Composition according to any one of the preceding claims, **characterized in that** it comprises at least one oil.

13. Composition according to any one of the preceding claims, **characterized in that** it comprises at least one volatile oil.

14. Composition according to the preceding claim, **characterized in that** the volatile oil is chosen from volatile hydrocarbon oils having from 8 to 16 carbon atoms; linear or cyclic silicone oils having from 2 to 7 silicon atoms, these silicones optionally comprising alkyl or alkoxy groups having from 1 to 10 carbon atoms; or volatile fluorinated oils.

15. Composition according to Claim 13 or 14, **characterized in that** the volatile oil is chosen from isododecane, isodecane, isohexadecane, octamethylcyclotetrasiloxane, decamethylcyclopentasiloxane, dodecamethylcyclohexasiloxane, heptamethylhexyltrisiloxane, heptamethyloctyltrisiloxane, hexamethyldisiloxane, octamethyltrisiloxane, decamethyltetrasiloxane or dodecamethylpentasiloxane.

16. Composition according to any one of Claims 13 to 15, **characterized in that** the volatile oil is present in a content ranging from 1% to 60% by weight with respect to the total weight of the composition, preferably ranging from 10% to 55% by weight, preferentially ranging from 15% to 50% by weight and more preferentially ranging from 20% to 50% by weight.

17. Composition according to any one of the preceding claims, **characterized in that** it comprises a non-volatile oil.

18. Composition according to the preceding claim, **characterized in that** the non-volatile oil is chosen from non-volatile silicone oils, non-volatile hydrocarbon oils, non-volatile fluorinated oils and their mixtures.

19. Composition according to Claim 17 or 18, **characterized in that** the non-volatile oil is a non-volatile silicone oil.

20. Composition according to any one of Claims 17 to 19, **characterized in that** the non-volatile oil is chosen from non-volatile polydimethylsiloxanes; polydimethylsiloxanes comprising pendant alkyl, alkoxy or phenyl groups or alkyl, alkoxy or phenyl groups at the end of the silicone chain, which groups have from 2 to 24 carbon atoms; phenylated silicones; polysiloxanes modified by fatty acids (in particular $C_8$-$C_{20}$ fatty acids), fatty alcohols (in particular $C_8$-$C_{20}$ fatty alcohols) or polyoxyalkylenes (in particular polyoxyethylene and/or polyoxypropylene); aminated silicones; silicones comprising hydroxyl groups; fluorosilicones comprising a pendant fluorinated group or a fluorinated group at the end of the silicone chain having from 1 to 12 carbon atoms, all or part of the hydrogens of which are substituted by fluorine atoms; and their mixtures.

21. Composition according to any one of Claims 17 to 20, **characterized in that** the non-volatile oil is chosen from liquid paraffin, squalane, hydrogenated polyisobutylene (Parleam oil), perhydrosqualene, mink, turtle, soybean, sweet almond, calophyllum, palm, grape seed, sesame, maize, arara, rapeseed, sunflower, cottonseed, apricot, castor, avocado, jojoba, olive or cereal germ oil; $C_{12}$-$C_{36}$ fatty esters; higher $C_{14}$-$C_{22}$ fatty acids; higher $C_{16}$-$C_{22}$ fatty alcohols; and their mixtures.

22. Composition according to any one of Claims 17 to 21, **characterized in that** the non-volatile oil is present in a content ranging from 1% to 60% by weight with respect to the total weight of the composition and preferably ranging from 5% to 50% by weight.

23. Composition according to any one of the preceding claims, **characterized in that** it comprises water in a content ranging from 10% to 80% by weight with respect to the total weight of the composition, preferably from 15% to 60% by weight, more preferentially from 20% to 50% by weight.

24. Composition according to any one of the preceding claims, **characterized in that** the aqueous phase comprises a solvent chosen from ethanol, isopropanol, glycerol, propylene glycol, butylene glycol, hexylene glycol, dipropylene glycol, diethylene glycol, mono-, di- or tripropylene glycol ($C_1$-$C_4$)alkyl ethers, mono-, di- or triethylene glycol ($C_1$-$C_4$) alkyl ethers and their mixtures.

25. Composition according to any one of the preceding claims, **characterized in that** the aqueous phase is present in a content ranging from 10% to 80% by weight with respect to the total weight of the composition, preferably from 15% to 60% by weight and more preferentially from 20% to 50% by weight.

26. Composition according to any one of the preceding claims, **characterized in that** it comprises a surfactant.

27. Composition according to any one of the preceding claims, **characterized in that** the composition is provided in the form of an oil-in-water emulsion or of a water-in-oil emulsion.

28. Composition according to any one of the preceding claims, **characterized in that** it comprises additional fillers other than the said particles dispersed in the aqueous phase and in the oily phase.

29. Composition according to the preceding claim, **characterized in that** the additional fillers are chosen from talc, mica, silica, kaolin, polyamide powders, poly-β-alanine powders, polyethylene powders, powders formed of tetrafluoroethylene polymers, lauroyllysine, starch, boron nitride, hollow microspheres of poly(vinylidene chloride/acrylonitrile), hollow microspheres of acrylic acid copolymers, silicone resin microbeads, particles formed of polyorganosiloxane elastomers, precipitated calcium carbonate, magnesium carbonate, basic magnesium carbonate, hydroxyapatite, hollow silica microspheres, porous silicas, ceramic microcapsules or metal soaps derived from organic carboxylic acids having from 8 to 22 carbon atoms.

30. Composition according to Claim 28 or 29, **characterized in that** the additional fillers are present in a content ranging from 0.1% to 25% by weight with respect to the total weight of the composition, preferably ranging from 1% to 20% by weight and more preferentially ranging from 5% to 15% by weight.

31. Composition according to any one of the preceding claims, **characterized in that** it comprises a colouring material.

32. Composition according to the preceding claim, **characterized in that** the colouring material is chosen from pigments, pearlescent agents, dyes and their mixtures.

33. Composition according to any one of the preceding claims, **characterized in that** the colouring material is present in a content ranging from 0.001% to 30% by weight with respect to the total weight of the composition, preferably ranging from 0.1% to 20% by weight and preferentially ranging from 1% to 15% by weight.

34. Composition according to any one of the preceding claims, **characterized in that** it comprises a cosmetic ingredient chosen from hydrophilic or lipophilic gelling and/or thickening agents, antioxidants, fragrances, preservatives, neutralizing agents, sunscreen agents, vitamins, moisturizing agents, self-tanning compounds, antiwrinkle active principles, emollients, hydrophilic or lipophilic active principles, agents for combating free radicals, sequestering agents, film-forming agents and their mixtures.

35. Non-therapeutic process for making up or caring for the skin comprising the application, to the skin, of a cosmetic composition according to any one of the preceding claims.

36. Use of a composition according to Claims 1 to 34 for softening wrinkles and/or giving a firm and tonic appearance to the skin.

**EP 1 671 614 B1**

37. Use of a composition according to Claims 1 to 34 for producing a make-up for the skin which softens wrinkles and/or which confers a firm and tonic appearance on the skin.


**Patentansprüche**

1. Kosmetische Zusammensetzung zum Schminken oder zur Pflege der Haut in der Form einer Emulsion, die ein wässrige Phase und eine Ölphase enthält, **dadurch gekennzeichnet, dass** sie eine Dispersion von Wachspartikeln mit einer volumenmittleren Größe von 1 $\mu$m oder darunter in der wässrigen Phase und in der Ölphase dispergierte Partikel mit einer volumenmittleren Größe von 15 $\mu$m oder darunter enthält.

2. Kosmetische Zusammensetzung zum Schminken oder zur Pflege der Haut in der Form einer Emulsion, die ein wässrige Phase und eine Ölphase enthält, **dadurch gekennzeichnet, dass** sie eine Dispersion einer Verbindung in Form von Partikeln mit einer volumenmittleren Größe von 1 $\mu$m oder darunter in der wässrigen Phase und eine Dispersion von Partikeln der gleichen Verbindung mit einer volumenmittleren Größe von 50 $\mu$m oder darunter in der Ölphase enthält.

3. Zusammensetzung nach dem vorhergehenden Anspruch, **dadurch gekennzeichnet, dass** die Partikel mit einer volumenmittleren Größe von 1 $\mu$m oder darunter, die in der wässrigen Phase dispergiert sind, unter den Wachsen, anorganischen Verbindungen und deren Gemischen ausgewählt sind.

4. Zusammensetzung nach dem vorhergehenden Anspruch, **dadurch gekennzeichnet, dass** die anorganischen Verbindungen unter den Kieselsäuren, Aluminiumoxiden oder deren Gemischen ausgewählt sind.

5. Kosmetische Zusammensetzung zum Schminken oder zur Pflege der Haut in der Form einer Emulsion, die ein wässrige Phase und eine Ölphase enthält, **dadurch gekennzeichnet, dass** sie Partikel mit einer volumenmittleren Größe von 1 $\mu$m oder darunter, die in der wässrigen Phase dispergiert sind und die unter den Wachsen, Kieselsäurepartikeln und deren Gemischen ausgewählt sind, und in der Ölphase dispergierte Partikel enthält, die unter den Wachspulvern, Kieselsäurepulvern und deren Gemischen ausgewählt sind.

6. Zusammensetzung nach dem vorhergehenden Anspruch, **dadurch gekennzeichnet, dass** sie Wachspartikel mit einer volumenmittleren Größe von 1 $\mu$m oder darunter, die in der wässrigen Phase dispergiert sind, und ein in der Ölphase dispergiertes Wachspulver enthält.

7. Zusammensetzung nach einem der Ansprüche 1 und 3 bis 6, **dadurch gekennzeichnet, dass** die Wachsdispersion in der wässrigen Phase Wachspartikel enthält, deren volumenmittlere Größe kleiner oder gleich 0,5 $\mu$m ist.

8. Zusammensetzung nach einem der Ansprüche 1 und 3 bis 7, **dadurch gekennzeichnet, dass** das Wachs unter Carnaubawachs, Candellilawachs, Ouricurywachs, Japanwachs, Korkfaserwachs, Zuckerrohrwachs, Ceresin, Paraffinwachsen, Lignitwachs, mikrokristallinen Wachsen, Lanolinwachs, Montanwachs, Ozokeriten, hydrierten Ölen, wie Jojobaöl, das hydriert ist, oder Ethylencopolymerisate, durch Fischer-Tropsch-Synthese hergestellten Wachsen, Fettsäureestern und Glyceriden, die bei 45 °C fest sind, Siliconwachsen, wie Alkylderivaten, Alkoxyderivaten und/oder Estern von Poly(di)methylsiloxan, die bei 45 °C fest sind und 10 bis 45 Kohlenstoffatome aufweisen, und verschiedenen Fettsäuren, wie Stearinsäure, Myristinsäure oder Behensäure, und deren Gemischen ausgewählt ist.

9. Zusammensetzung nach einem der Ansprüche 1 und 3 bis 8, **dadurch gekennzeichnet, dass** das in der wässrigen Phase dispergierte Wachs in einem Mengenanteil im Bereich von 0,01 bis 15 Gew.-%, bezogen auf das Gesamtgewicht der Zusammensetzung, vorzugsweise im Bereich von 0,5 bis 10 Gew.-%, bevorzugt im Bereich von 1 bis 8 Gew.-% und besonders bevorzugt im Bereich von 1 bis 7 Gew.-% enthalten ist.

10. Kosmetische Zusammensetzung nach einem der Ansprüche 2 bis 9, **dadurch gekennzeichnet, dass** die in der Ölphase dispergierten Partikel eine volumenmittlere Größe von kleiner oder gleich 30 $\mu$m, vorzugsweise von kleiner oder gleich 15 $\mu$m und noch bevorzugter von kleiner oder gleich 10 $\mu$m aufweisen.

11. Zusammensetzung nach einem der vorhergehenden Ansprüche, **dadurch gekennzeichnet, dass** die in der Ölphase dispergierten Partikel in einem Mengenanteil von 0,1 bis 15 Gew.-%, bezogen auf das Gesamtgewicht der Zusammensetzung, vorzugsweise im Bereich von 0,5 bis 10 Gew.-%, und besonders bevorzugt im Bereich von 0,5 bis 8 Gew.-% enthalten sind.

**16**

**12.** Zusammensetzung nach einem der vorhergehenden Ansprüche, **dadurch gekennzeichnet, dass** sie mindestens ein Öl enthält.

**13.** Zusammensetzung nach einem der vorhergehenden Ansprüche, **dadurch gekennzeichnet, dass** sie mindestens ein flüchtiges Öl enthält.

**14.** Zusammensetzung nach dem vorhergehenden Anspruch, **dadurch gekennzeichnet, dass** das flüchtige Öl unter den flüchtigen Kohlenwasserstoffölen mit 8 bis 16 Kohlenstoffatomen; linearen oder cyclischen Siliconölen mit 2 bis 7 Siliciumatomen, wobei diese Silicone gegebenenfalls Alkyl- oder Alkoxygruppen mit 1 bis 10 Kohlenstoffatomen aufweisen; und fluorierten flüchtigen Ölen ausgewählt ist.

**15.** Zusammensetzung nach Anspruch 13 oder 14, **dadurch gekennzeichnet, dass** das flüchtige Öl unter Isododecan, Isodecan, Isohexadecan, Octamethylcyclotetrasiloxan, Decamethylcyclopentasiloxan, Dodecamethylcyclohexasiloxan, Heptamethylhexyltrisiloxan, Heptamethyloctyltrisiloxan, Hexamethyldisiloxan, Octamethyltrisiloxan, Decamethyltetrasiloxan und Dodecamethylpentasiloxan ausgewählt ist

**16.** Zusammensetzung nach einem der Ansprüche 13 bis 15, **dadurch gekennzeichnet, dass** das flüchtige Öl in einem Mengenanteil im Bereich von 1 bis 60 Gew.-%, bezogen auf das Gesamtgewicht der Zusammensetzung, vorzugsweise im Bereich von 10 bis 55 Gew.-%, bevorzugt im Bereich von 15 bis 50 Gew.- % und besonders bevorzugt im Bereich von 20 bis 50 Gew.-% enthalten ist.

**17.** Zusammensetzung nach einem der vorhergehenden Ansprüche, **dadurch gekennzeichnet, dass** sie ein nicht flüchtiges Öl enthält.

**18.** Zusammensetzung nach dem vorhergehenden Anspruch, **dadurch gekennzeichnet, dass** das nicht flüchtige Öl unter den nicht flüchtigen Siliconölen, nicht flüchtigen Kohlenwasserstoffölen, nicht flüchtigen fluorierten Ölen und deren Gemischen ausgewählt ist.

**19.** Zusammensetzung nach Anspruch 17 oder 18, **dadurch gekennzeichnet, dass** das nicht flüchtige Öl ein nicht flüchtiges Siliconöl ist.

**20.** Zusammensetzung nach einem der Ansprüche 17 bis 19, **dadurch gekennzeichnet, dass** das nicht flüchtige Öl unter den nicht flüchtigen Polydimethylsiloxanen; Polydimethylsiloxanen, die als Seitenkette oder am Ende der Siliconkette Alkyl-, Alkoxy- oder Phenylgruppen tragen, wobei diese Gruppen 2 bis 24 Kohlenstoffatome aufweisen; phenylierten Siliconen; Polysiloxanen, die mit Fettsäuren (insbesondere mit 8 bis 20 Kohlenstoffatomen), mit Fettalkoholen (insbesondere mit 8 bis 20 Kohlenstoffatomen) oder Polyoxyalkylenen (besonders Polyoxyethylen und/ oder Polyoxypropylen) modifiziert sind; aminierten Siliconen; Siliconen mit Hydroxygruppen; fluorierten Siliconen, die als Seitenkette oder am Ende der Siliconkette eine fluorierte Gruppe mit 1 bis 12 Kohlenstoffatomen tragen, bei der ein Teil der Wasserstoffatome oder alle Wasserstoffatome durch Fluoratome ersetzt sind; und deren Gemischen ausgewählt ist.

**21.** Zusammensetzung nach einem der Ansprüche 17 bis 20, **dadurch gekennzeichnet, dass** das nicht flüchtige Öl ausgewählt ist unter: Paraffinöl, Squalan, hydriertem Polyisobutylen (Parleamöl), Perhydrosqualen, Nerzöl, Schildkrötenöl, Sojaöl, Süßmandelöl, Calophyllumöl, Palmöl, Traubenkernöl, Sesamöl, Maisöl, Araraöl, Rapsöl, Sonnenblumenöl, Baumwollsamenöl, Aprikosenkernöl, Ricinusöl, Avocadoöl, Jojobaöl, Olivenöl oder Getreidekeimölen; Fettsäureestern mit 12 bis 36 Kohlenstoffatomen; höheren Fettsäuren mit 14 bis 22 Kohlenstoffatomen; höheren Fettalkoholen mit 16 bis 22 Kohlenstoffatomen; und deren Gemischen.

**22.** Zusammensetzung nach einem der Ansprüche 17 bis 21, **dadurch gekennzeichnet, dass** das nicht flüchtige Öl in einem Mengenanteil von 1 bis 60 Gew.-%, bezogen auf das Gesamtgewicht der Zusammensetzung, und vorzugsweise im Bereich von 5 bis 50 Gew.-% enthalten ist.

**23.** Zusammensetzung nach einem der vorhergehenden Ansprüche, **dadurch gekennzeichnet, dass** sie Wasser in einem Mengenanteil von 10 bis 80 Gew.-%, bezogen auf das Gesamtgewicht der Zusammensetzung, vorzugsweise 15 bis 60 Gew.-% und noch bevorzugter 20 bis 50 Gew.-% enthält.

**24.** Zusammensetzung nach einem der vorhergehenden Ansprüche, **dadurch gekennzeichnet, dass** die wässrige Phase eine Lösemittel enthält, das ausgewählt ist unter Ethanol, Isopropanol, Glycerol, Propylenglycol, Butylenglycol, Hexylenglycol, Dipropylenglycol, Diethylenglycol, Alkyl($C_{1-4}$)ethern von Mono-, Di- oder Tripropylenglycol, Alkyl ($C_{1-4}$)ethern von Mono-, Di- oder Triethylenglycol und deren Gemischen.

25. Zusammensetzung nach einem der vorhergehenden Ansprüche, **dadurch gekennzeichnet, dass** die wässrige Phase in einem Mengenanteil von 10 bis 80 Gew.-%, bezogen auf das Gesamtgewicht der Zusammensetzung, vorzugsweise 15 bis 60 Gew.-% und noch bevorzugter 20 bis 50 Gew.-% enthalten ist.

26. Zusammensetzung nach einem der vorhergehenden Ansprüche, **dadurch gekennzeichnet, dass** sie einen grenzflächenaktiven Stoff enthält.

27. Zusammensetzung nach einem der vorhergehenden Ansprüche, **dadurch gekennzeichnet, dass** die Zusammensetzung in Form einer Öl-in-Wasser-Emulsion oder Wasser-in-Öl-Emulsion vorliegt.

28. Zusammensetzung nach einem der vorhergehenden Ansprüche, **dadurch gekennzeichnet, dass** die ergänzende Füllstoffe enthält, die von den in der wässrigen Phase und in der Ölphase dispergierten Partikeln verschieden sind.

29. Zusammensetzung nach dem vorhergehenden Anspruch, **dadurch gekennzeichnet, dass** die ergänzenden Füllstoffe unter Talk, Glimmer, Kieselsäure, Kaolin, Polyamidpulvern, Poly-βalaninpulvern, Polyethylenpulvern, Pulvern von Tetrafluorethylenpolymeren, Lauroyllysin, Stärke, Bornitrid, Mikrohohlkugeln aus Polyvinylidenchlorid/Acrylnitril, Mikrohohlkugeln von Acrylsäurecopolymeren, Siliconharzmikrokugeln, Partikeln von elastomeren Polyorganosiloxanen, gefälltem Calciumcarbonat, Magnesiumcarbonat, Magnesiumhydrogencarbonat, Hydroxyapatit, Siliciumoxid-Mikrohohlkugeln, porösen Kieselsäuren, Keramikmikrokapseln und Metallseifen, die von organischen Carbonsäuren mit 8 bis 22 Kohlenstoffatomen abgeleitet sind, ausgewählt ist.

30. Zusammensetzung nach Anspruch 28 oder 29, **dadurch gekennzeichnet, dass** die ergänzenden Füllstoffe in einem Mengenanteil von 0,1 bis 25 Gew.-%, bezogen auf das Gesamtgewicht der Zusammensetzung, vorzugsweise 1 bis 20 Gew.-% und noch bevorzugter 5 bis 15 Gew.-% enthalten sind.

31. Zusammensetzung nach einem der vorhergehenden Ansprüche, **dadurch gekennzeichnet, dass** sie ein Farbmittel enthält.

32. Zusammensetzung nach dem vorhergehenden Anspruch, **dadurch gekennzeichnet, dass** das Farbmittel unter den Pigmenten, Perlglanzpigmenten, Farbstoffen und deren Gemischen ausgewählt ist.

33. Zusammensetzung nach einem der vorhergehenden Ansprüche, **dadurch gekennzeichnet, dass** das Farbmittel in einem Mengenanteil im Bereich von 0,001 bis 30 Gew.-%, bezogen auf das Gesamtgewicht der Zusammensetzung, vorzugsweise im Bereich von 0,1 bis 20 Gew.-% und bevorzugt im Bereich von 1 bis 15 Gew.-% vorliegt.

34. Zusammensetzung nach einem der vorhergehenden Ansprüche, **dadurch gekennzeichnet, dass** sie einen kosmetischen Bestandteil enthält, der unter den hydrophilen oder lipophilen Gelbildnern und/ oder Verdickungsmitteln, Antioxidantien, Parfums, Konservierungsmitteln, Neutralisationsmitteln, Sonnenschutzfiltern, Vitaminen, Hydratisierungsmitteln, Selbstbräunungsmitteln, Wirkstoffen gegen Falten, Emollientien, hydrophilen oder lipophilen Wirkstoffen, Radikalfängern für freie Radikale, Maskierungsmitteln, Filmbildnern und deren Gemischen ausgewählt ist.

35. Nicht therapeutisches Verfahren zum Schminken oder zur Pflege der Haut, das das Auftragen einer kosmetischen Zusammensetzung nach einem der vorhergehenden Ansprüche auf die Haut umfasst.

36. Verwendung einer Zusammensetzung nach einem der Ansprüche 1 bis 34, um Falten zu kaschieren und/oder der Haut ein festes und straffes Aussehen zu geben.

37. Verwendung einer Zusammensetzung nach einem der Ansprüche 1 bis 34, um auf der Haut eine Schminke zu bilden, die Falten kaschiert und/oder der Haut ein festes und straffes Aussehen gibt.

EP 1 671 614 B1

RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 1099437 A

- EP 1086683 A